# FASCICULE DE BREVET EUROPEEN

(11) **EP 3 378 068 B1**
(45) Date de publication et mention de la délivrance du brevet: **21.10.2020**
(21) Numéro de dépôt: 16798682.7
(22) Date de dépôt: 15.11.2016
(51) Int. Cl.: G21F 5/015, A61M 5/14, G21F 1/08, A61M 5/00

(54) **DISPOSITIF D'ADMINISTRATION D'UN MEDICAMENT RADIO-PHARMACEUTIQUE**
VORRICHTUNG ZUR VERABREICHUNG EINES RADIOPHARMAKONS
DEVICE FOR ADMINISTERING A RADIOPHARMACEUTICAL DRUG

(30) Priorité: 16.11.2015 FR 1560996
(43) Date de publication de la demande: 26.09.2018
(73) Titulaire: Assistance Publique - Hôpitaux de Paris, 75004 Paris (FR)
(72) Inventeur: STARZ, Christian, 95430 Butry-sur-oise (FR); DARRAS, Philippe, 89390 Aisy-sur-armencon (FR); CARADEC, Hervé, 77420 Champs-sur-marne (FR); BEN REGUIGA, Makrem, 78230 Le Pecq (FR)
(74) Mandataire: Nony
(86) Numéro de dépôt international: PCT/EP2016/077657
(87) Numéro de publication internationale: WO 2017/085038

(56) Documents cités:
- WO-A1-2014/188401
- FR-A- 1 264 753
- US-A- 2 533 102
- US-A1- 2011 124 948

## Description

### Domaine Technique

La présente invention concerne un dispositif d'administration d'un médicament radio-pharmaceutique destiné à être suspendu à un pied à perfusion, notamment pour une injection en intraveineuse. L'invention concerne également un pied à perfusion pourvu d'un tel dispositif.

### Art antérieur

Les médicaments radio-pharmaceutiques destinés à la radiothérapie métabolique sont généralement administrés par perfusion en intraveineuse lente. Ces médicaments, classiquement livrés dans un conteneur pourvu d'un bouclier radio-opaque, peuvent être transférés dans une seringue, puis administrés avec un pousse-seringue. Cette procédure expose cependant le personnel à des risques d'irradiation.

L'objectif de la présente invention est de fournir un dispositif d'administration permettant de limiter ce risque.

Des documents pertinents de l'état de la technique sont : US 2011/124948 A1, FR 1.264.753 A, US 2.533.102A, et WO2014/188401 A1.

### Résumé de l'invention

L'invention propose un dispositif d'administration comportant
- un conteneur hermétique fermé par un bouchon, contenant un médicament radio-pharmaceutique, et pourvu d'un bouclier de protection en un matériau radio-opaque, et
- un support pourvu de moyens d'accrochage, le conteneur étant fixé au support, de manière amovible, dans une position autorisant un accès audit bouchon.

Le dispositif d'administration inventif est caractérisé en ce que le support comporte un socle pourvu d'une ouverture autorisant un accès audit bouchon, un couvercle et des tiges reliant le socle et le couvercle, le conteneur s'étendant entre le couvercle et le socle.

Comme on le verra plus en détails dans la suite de la description, le dispositif peut être facilement manipulé et installé sur un pied à perfusion. En outre, l'accès au conteneur est possible à travers le support, ce qui facilite la mise en place de la perfusion. Enfin, l'exposition du personnel aux émissions radioactives est limitée.

Un dispositif d'administration selon l'invention peut encore comporter une ou plusieurs des caractéristiques optionnelles suivantes :
- le dispositif comporte un unique conteneur ;
- le bouclier de protection comporte du plomb et/ou du tungstène ;
- les moyens d'accrochage comportent un anneau ;
- le conteneur comporte un récipient, de préférence un unique récipient, d'épaisseur constante ;
- les moyens d'accrochage sont fixés sur un couvercle du support ;
- le médicament radio-pharmaceutique est adapté à la radiothérapie métabolique ou à une application diagnostique ;
- au moins une extrémité d'une desdites tiges, de référence de chacune desdites tiges, est désolidarisable du couvercle ou du support sur lequel elle est fixée, de préférence manuellement ;
- le conteneur s'étend dans l'espace entre le couvercle, le socle et les tiges ;
- le support comporte un couvercle pourvu de trous traversés par des tiges fixées sur le socle ;
- les tiges comportent des butées sur lesquelles le couvercle prend appui par gravité ;
- au moins une, de préférence chaque tige, présente à son extrémité libre, une portion filetée qui fait saillie au-dessus du couvercle et sur laquelle un boulon est vissé de manière à être en appui sur le couvercle ;
- le dispositif comporte un cylindre, de préférence transparent, reposant sur le support, et dans lequel le conteneur est logé ;
- la hauteur du cylindre est déterminée de manière que le fond du conteneur prenne appui sur le couvercle.

L'invention propose également un appareil à perfusion comportant un pied à perfusion et un dispositif d'administration selon l'invention suspendu audit pied.

Le pied à perfusion peut comporter des roulettes, et de préférence, des moyens de blocage desdites roulettes.

L'appareil à perfusion peut encore comporter un dispositif d'injection comportant une aiguille d'injection et un cathéter mettant en relation de fluide le médicament radio-pharmaceutique sortant du conteneur et ladite aiguille d'injection. Le dispositif d'injection comporte de préférence une canule de perfusion connectée au cathéter et traversant le bouchon fermant le conteneur.

### Brève description des figures

D'autres caractéristiques et avantages de l'invention apparaîtront encore à la lecture de la description détaillée qui va suivre, et à l'examen du dessin annexé, dans lequel:
- la figure 1 représente, en perspective, un dispositif d'administration dans un mode de réalisation préféré de l'invention ;
- la figure 2 représente le support du dispositif de la figure 1 démonté, le cylindre n'étant pas représenté ;
- la figure 3 représente le dispositif de la figure 1 vu de côté ; et
- les figures 4 et 5 représentent, en coupes transversales médianes, le couvercle et le socle du support du dispositif de la figure 1.

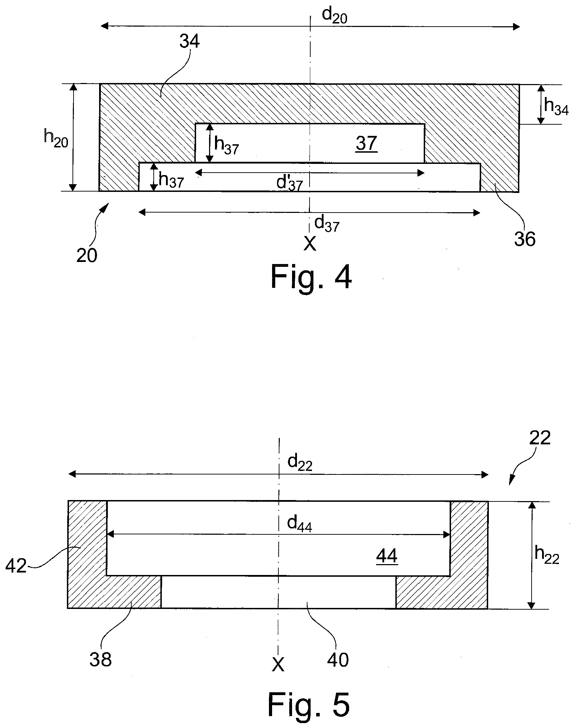

Dans un souci de simplification, l'anneau 30 de suspension n'a pas été représenté sur la figure 4.

### Définitions

Un matériau « radio-opaque » est un matériau apte à filtrer et/ou atténuer, au moins partiellement, le rayonnement radioactif émis par le médicament radio-pharmaceutique, et en particulier les rayonnements photoniques ou particulaires.

Sauf indication contraire, « comportant», « comprenant », « ayant », « incluant » ou leurs variations correspondent à une inclusion non exclusive.

La description des figures est faite en référence à une direction verticale V. En particulier, les adjectifs "inférieur", "supérieur", "horizontal" et "vertical" font référence à cette direction.

« Transversal » fait référence à une direction ou à un plan perpendiculaire à l'axe X du dispositif.

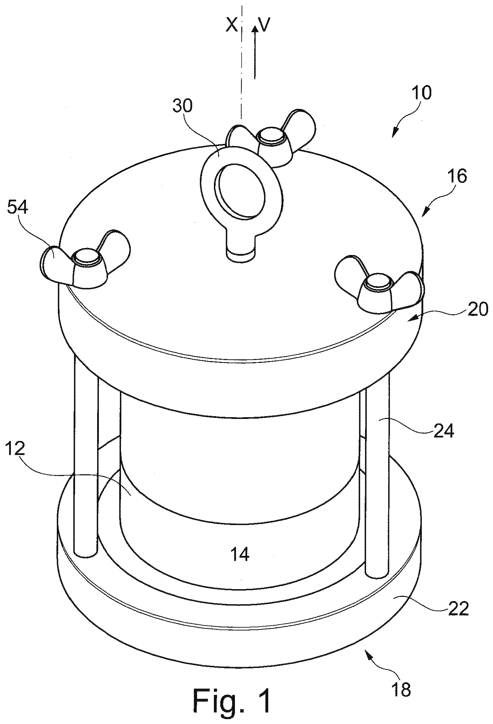

Sauf indication contraire, les pièces sont décrites dans la position assemblée, comme représenté sur la figure 1.

### Description détaillée

Comme illustré sur les figures, un dispositif d'administration 10 selon l'invention comporte un conteneur 12 contenant un médicament radio-pharmaceutique 14, et un support 16 portant le conteneur 12. Le support 16 comprend une base 18 et un couvercle 20. La base 18 est constituée d'un socle 22 et de trois tiges 24.

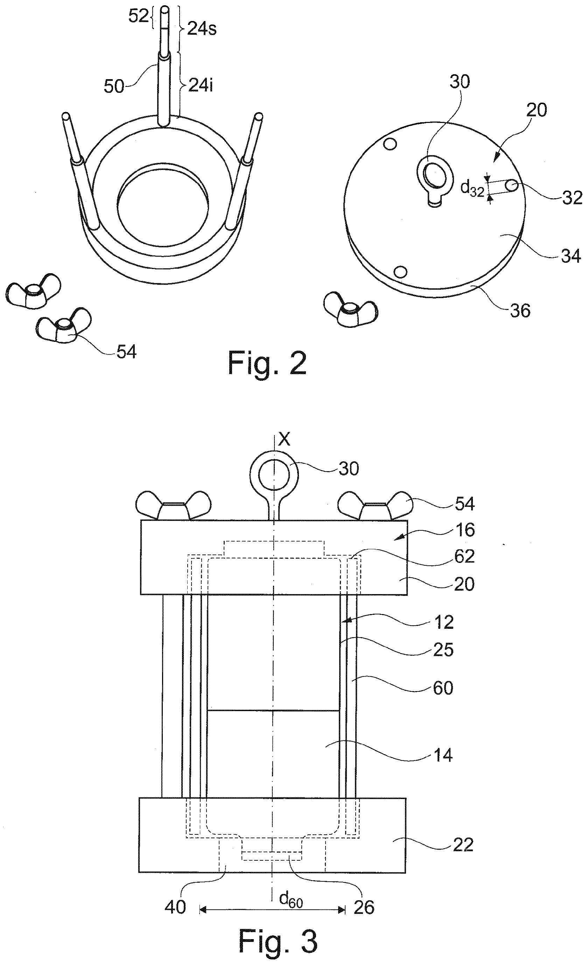

Le conteneur 12 est un conteneur classiquement utilisé pour contenir un médicament radio-pharmaceutique. Plus précisément, il comporte un récipient 25 fermé par un bouchon 26 et pourvu d'un bouclier de protection en un matériau radio-opaque. Dans la position de service représentée sur la figure 1 ou sur la figure 3, le conteneur 12 est disposé à l'envers, le bouchon étant vers le bas, afin que le médicament puisse s'écouler par gravité dans une canule, non représentée, traversant le bouchon.

Le bouclier de protection radio-opaque, encore appelé aussi « pot plombé » ou « protège-flacon », peut être, par exemple, en plomb, en tungstène, en un verre au plomb, en un sandwich plomb/PMMA (Plexiglas), en un sandwich tungstène/PMMA, ou en un mélange de ces constituants.

De préférence, le bouclier de protection est adapté pour filtrer plus de 50%, de préférence plus de 70%, de préférence plus de 80%, de préférence plus de 90%, de préférence plus de 95%, de préférence sensiblement 100% du rayonnement radioactif émis par le médicament radio -pharmaceutique 14.

Le médicament radio-pharmaceutique 14 peut être en particulier adapté à la radiothérapie métabolique. Le médicament radio-pharmaceutique peut être en particulier du Quadramet®, du Métatstron®, du MIBG thérapeutique®, MIBG Diagnostique®, Adreview®, ou du Lutathéra®.

Le médicament radio-pharmaceutique 14 peut être adapté à une application diagnostique.

Comme représenté sur la figure 2, le couvercle 20 comporte, en partie supérieure, sensiblement en son centre, des moyens d'accrochage, par exemple un crochet, de préférence un anneau 30, adaptés pour une suspension du dispositif d'administration à un pied à perfusion.

Le couvercle 20 présente de préférence une forme générale de disque d'axe X vertical.

Le diamètre *d₂₀* du couvercle 20 est de préférence supérieur à 80 mm, de préférence supérieur à 90 mm, de préférence supérieur à 95 mm, et/ou inférieur à 110 mm, de préférence inférieur à 105 mm, de préférence d'environ 99 mm.

La hauteur *h₂₀* du couvercle 20 (mesurée selon l'axe vertical X) est de préférence supérieure à 15 mm, supérieure à 20 mm et/ou inférieure à 30 mm, inférieure à 25 mm, de préférence d'environ 23 mm.

Le couvercle 20 comporte de préférence une paroi supérieure 34 sensiblement horizontale dans la position assemblée représentée sur la figure 1, ceinturée par un rebord inférieur 36, s'étendant sensiblement verticalement sous la paroi supérieure 34.

La hauteur *h₃₄* de la paroi supérieure 34 est de préférence supérieure à 5 mm et/ou inférieure à 10 mm, de préférence d'environ 8 mm.

Le couvercle 20 est pourvu de trous 32, disposés à sa périphérie, le traversant selon son épaisseur, sensiblement verticaux, et équiangulairement répartis autour de l'axe X.

Chaque trou 32 présente un diamètre *d₃₂* légèrement supérieur à celui de la partie supérieure d'une tige 24. Ce diamètre est de préférence supérieur à 5 mm, supérieur à 6 mm, supérieur à 7 mm et/ou inférieur à 10 mm, inférieur à 9 mm, inférieur à 8 mm. Dans un mode de réalisation préféré, le diamètre de chacun des trous 32 est identique.

De préférence, les trous 32 sont ménagés à travers le rebord 36.

Le rebord 36 et la paroi supérieure 34 définissent une cuvette 37 qui présente de préférence une berge à profil crénelée, comme représenté sur la figure 4. Le profil de la cuvette 37 est de préférence de révolution autour de l'axe X.

Depuis le bas vers le haut, le diamètre intérieur de la cuvette peut en particulier être égal à un diamètre d₃₇ supérieur à 70 mm, de préférence supérieur à 75 mm et/ou inférieur à 90 mm, de préférence inférieur à 85 mm, de préférence d'environ 81 mm, puis égal à un diamètre *d'₃₇* supérieur à 45 mm, supérieur à 50 mm et/ou inférieur à 65 mm, inférieur à 60 mm, de préférence d'environ 55 mm.

La hauteur *h₃₇* d'au moins une, de préférence de chaque marche du profil crénelé est de préférence supérieure à 5 mm et inférieure à 10 mm, par exemple de 7 ou 8 mm.

Le conteneur 12 est posé sur le socle 22 de la base 18. Le socle 22 présente une forme générale annulaire, d'axe X.

Le diamètre *d₂₂* du socle 22 est de préférence supérieur à 80 mm, de préférence supérieur à 90 mm, de préférence supérieur à 95 mm, et/ou inférieur à 110 mm, de préférence inférieur à 105 mm, de préférence d'environ 99 mm. Il est de préférence sensiblement identique au diamètre extérieur *d₂₀* du couvercle.

La hauteur *h₂₂* du socle 22 (mesurée selon l'axe vertical X) est de préférence supérieure à 15 mm, supérieure à 20 mm et/ou inférieure à 30 mm, inférieure à 25 mm, de préférence d'environ 23 mm.

Comme représenté sur la figure 5, le socle 22 comporte un fond 38, percé d'une ouverture centrale 40, et un rebord supérieur 42 s'étendant en périphérie du fond 38 vers le haut, sensiblement selon l'axe X. L'ouverture 40 est adaptée de manière à autoriser un accès au conteneur 12, et plus précisément au bouchon 26 du conteneur 12, lorsque celui-ci est en appui sur le fond 38, comme représenté sur la figure 3. Avantageusement, il est ainsi facile de faire passer une canule de perfusion à travers ledit bouchon.

Le fond 38 et le rebord supérieur 42 définissent un logement 44 pour le conteneur 12. Le logement 44, de préférence sensiblement cylindrique, de section circulaire, présente un diamètre *d₄₄* de préférence sensiblement identique au plus grand diamètre d₃₇ de la cuvette 37 du couvercle.

Les tiges 24 sont fixées, sensiblement verticalement, sur le socle 22, de préférence dans le rebord supérieur 42. Elles peuvent être fixées par tout moyen, par exemple par vissage, soudage, insertion en force ou frettage.

Le nombre de tiges 24 et de trous 32 n'est pas limité. Il est de préférence supérieur à 2 et/ou inférieur à 5, de préférence inférieur à 4, de préférence de trois.

Les tiges 24 sont disposées autour de l'axe X de manière à pouvoir chacune traverser un trou 32 du couvercle. Elles sont de préférence équiangulairement réparties autour de l'axe X.

Dans un mode de réalisation, les tiges 24 sont sensiblement identiques.

De préférence, une tige 24 comporte une partie inférieure 24ᵢ dont le diamètre est supérieur au diamètre du trou 32 correspondant, et une partie supérieure 24ₛ présentant un diamètre inférieur au diamètre dudit trou 32. Avantageusement, la transition entre les parties inférieure 24ᵢ et supérieure 24ₛ définit un rebord formant une butée 50 pour le couvercle 20.

La partie supérieure 24ₛ comporte une portion filetée 52 terminale qui, dans la position de butée du couvercle sur la butée 50, fait au moins en partie saillie au-dessus de la paroi supérieure 34 du couvercle. Des boulons 54, de préférence des boulons à ailettes, peuvent alors être vissés sur la portion filetée 52 afin de serrer le couvercle contre la butée 50.

Le couvercle et/ou le socle et/ou les tiges peuvent être, par exemple, en acier ou en aluminium.

Dans un mode de réalisation, le dispositif d'administration comporte encore un cylindre 60, de préférence de section circulaire, qui, dans la position assemblée, s'étend sensiblement verticalement depuis le socle jusqu'au couvercle. Le cylindre 60 s'étend de préférence entre les tiges 24, comme représenté sur la figure 3.

Dans un mode de réalisation, le diamètre extérieur du cylindre 60 est sensiblement identique, mais légèrement inférieur, au diamètre *d₄₄* du logement 44 du socle 22 et/ou à un des diamètres intérieurs de la cuvette 37, ce qui permet d'éviter tout déplacement latéral du cylindre 60 dans la position assemblée.

Le diamètre intérieur *d₆₀* du cylindre 60 est de préférence adapté de manière que le cylindre 60 puisse recevoir un conteneur 12 tout en l'immobilisant latéralement. Autrement dit, la plus grande dimension transversale du conteneur 12 est de préférence légèrement inférieure au diamètre intérieur *d₆₀* du cylindre 60.

Si le cylindre dépasse le niveau de la butée 50, le couvercle prend appui sur le chant 62 supérieur du cylindre, et non plus sur la butée 50. La position, selon l'axe X, du couvercle 20 par rapport au socle 22 peut alors être modifiée par simple changement du cylindre 60. Bien entendu, la longueur de la portion filetée 52 doit être adaptée en conséquence.

L'invention concerne également un ensemble comportant un dispositif d'administration selon l'invention et plusieurs cylindres 60 présentant différentes hauteurs et/ou différents diamètres intérieurs *d₆₀.*

De préférence, ces hauteurs et diamètres intérieurs *d₆₀* sont adaptés à des hauteurs et diamètres extérieurs de conteneurs 12 commercialisés.

Le cylindre 60 présente de préférence une paroi radio-opaque.

De préférence, le cylindre 60 est transparent, de manière que l'on puisse apercevoir le conteneur 12, et notamment vérifier son niveau de remplissage.

Le fonctionnement du dispositif d'administration est le suivant, dans le mode de réalisation préféré.

Le dispositif est initialement dans la position démontée représentée sur la figure 2.

Un cylindre 60 présentant un diamètre intérieur légèrement supérieur à celui du conteneur 12 est posé sur le socle, entre les tiges 24. Il est immobilisé latéralement dans le logement 44.

Le conteneur 12 contenant le médicament radio-pharmaceutique est introduit, avec son bouclier radio-opaque, dans le cylindre 60 et posé, à l'envers, de manière à prendre appui, par gravité, sur le socle 22. Le diamètre intérieur du cylindre 60 est légèrement supérieur au diamètre extérieur du conteneur 12, ce qui permet de maintenir parfaitement en position le conteneur 12. Le bouchon est accessible à travers l'ouverture 40, comme représenté sur la figure 3.

Le couvercle 20 est alors disposé de manière que les tiges 24 soient introduites dans les trous 32, jusqu'à dépasser au-dessus de la paroi supérieure 34.

Les boulons à ailettes 54 sont alors vissés sur les tiges 24 de manière à pousser le couvercle 16 contre le cylindre 60 ou contre les butées 50, selon que le cylindre dépasse en hauteur ou non les butées 50.

La hauteur du cylindre est de préférence déterminée de manière que, dans la position assemblée représentée sur la figure 1, le fond du conteneur prenne appui sur le couvercle 20. Avantageusement, le conteneur ne recule donc pas vers le couvercle lorsque la canule de perfusion est introduite à travers le bouchon 26.

L'ouverture 40 permet avantageusement d'introduire une canule de perfusion à travers le bouchon 26 afin d'extraire le médicament. Le dispositif d'administration ainsi constitué peut être suspendu à un pied à perfusion par l'anneau 30.

Avantageusement, le médicament reste conditionné dans son emballage d'origine, à savoir le conteneur 12, ce qui évite tout risque d'irradiation du personnel. En outre, le conditionnement du conteneur 12 entre le couvercle 20 et le socle 22 est rapide et pratique. Enfin, il est efficacement maintenu en position entre le socle, le couvercle et le cylindre 60. Le dispositif est alors suspendu à un pied à perfusion par l'anneau 30.

Bien entendu, l'invention n'est pas limitée aux modes de réalisation décrits et représentés, fournis à titre d'exemples illustratifs seulement.

En particulier, les tiges pourraient être fixées sur le couvercle. Les tiges pourraient traverser des trous ménagés dans le socle et présenter, de préférence, une portion filetée à leur extrémité libre inférieure, un boulon étant vissé sur ladite portion filetée et le socle reposant sur ledit boulon.

## Revendications

1. Dispositif d'administration comportant
- un conteneur (12) hermétique fermé par un bouchon (26), contenant un médicament radio-pharmaceutique (14), et pourvu d'un bouclier de protection en un matériau radio-opaque, et
- un support (16) pourvu de moyens d'accrochage (30), le conteneur (12) étant fixé au support, de manière amovible, dans une position autorisant un accès audit bouchon,
**caractérisé en ce que** le support comporte un socle (22) pourvu d'une ouverture (40) autorisant un accès audit bouchon, un couvercle (20) et des tiges reliant le socle et le couvercle,
le conteneur s'étendant entre le couvercle et le socle.

2. Dispositif selon la revendication précédente, dans lequel le bouclier de protection comporte du plomb et/ou du tungstène.

3. Dispositif selon l'une quelconque des revendications précédentes, dans lequel les moyens d'accrochage comportent un anneau.

4. Dispositif selon l'une quelconque des revendications précédentes, dans lequel le couvercle (20) est pourvu de trous (32) traversés par les tiges (24), les tiges étant fixées sur le socle (22).

5. Dispositif selon l'une quelconque des revendications précédentes, dans lequel les tiges comportent des butées (50) sur lesquelles le couvercle prend appui par gravité.

6. Dispositif selon l'une quelconque des revendications précédentes, dans lequel au moins une, de préférence chaque tige (24), présente à son extrémité libre, une portion filetée (52) qui fait saillie au-dessus du couvercle et sur laquelle un boulon (54) est vissé de manière à être en appui sur le couvercle.

7. Dispositif selon l'une quelconque des revendications précédentes, comportant un cylindre (60), reposant sur le support, et dans lequel le conteneur est logé.

8. Dispositif selon la revendication immédiatement précédente, dans lequel la hauteur du cylindre est déterminée de manière que le fond du conteneur prenne appui sur le couvercle.

9. Dispositif selon l'une quelconque des deux revendications immédiatement précédentes, dans lequel le cylindre est transparent, de manière que l'on puisse apercevoir le conteneur.

10. Appareil à perfusion comportant un pied à perfusion et un dispositif d'administration selon l'une quelconque des revendications précédentes suspendu audit pied.

11. Ensemble comportant
- un dispositif d'administration selon l'une des revendications précédentes et
- plusieurs cylindres présentant différentes hauteurs et/ou différents diamètres intérieurs et adaptés pour pouvoir être serrés en sandwich entre le socle et le couvercle du support.

## Patentansprüche

1. Verabreichungsvorrichtung aufweisend
- einen hermetischen Behälter (12), der durch einen Stopfen (26) verschlossen ist, der ein Radiopharmakon (14) enthält und mit einer Schutzabschirmung aus einem strahlenundurchlässigen Material versehen ist, und
- einen Träger (16), der mit Aufhängemitteln (30) versehen ist, wobei der Behälter (12) abnehmbar in einer Position an dem Träger befestigt ist, die einen Zugang zu dem Stopfen gestattet,
**dadurch gekennzeichnet, dass** der Träger einen Sockel (22), der mit einer Öffnung (40) versehen ist, die einen Zugang zu dem Stopfen gestattet, einen Deckel (20) und Stangen, die den Sockel und den Deckel verbinden, aufweist, wobei sich der Behälter zwischen dem Deckel und dem Sockel erstreckt.

2. Vorrichtung nach dem vorhergehenden Anspruch, wobei die Schutzabschirmung Blei und/oder Wolfram aufweist.

3. Vorrichtung nach einem der vorhergehenden Ansprüche, wobei die Aufhängemittel einen Ring umfassen.

4. Vorrichtung nach einem der vorhergehenden Ansprüche, wobei der Deckel (20) mit Löchern (32) versehen ist, die von den Stangen (24) durchquert werden, wobei die Stangen auf dem Sockel (22) befestigt sind.

5. Vorrichtung nach einem der vorhergehenden Ansprüche, wobei die Stangen Anschläge (50) aufweisen, an denen der Deckel durch Schwerkraft anliegt.

6. Vorrichtung nach einem der vorhergehenden Ansprüche, wobei mindestens eine, vorzugsweise jede Stange (24) an ihrem freien Ende einen Gewindeabschnitt (52) aufweist, der über den Deckel hervorsteht und auf den ein Bolzen (54) so geschraubt ist, dass er an dem Deckel anliegt.

7. Vorrichtung nach einem der vorhergehenden Ansprüche, aufweisend einen Zylinder (60), der auf dem Träger aufliegt und in dem der Behälter aufgenommen ist.

8. Vorrichtung nach dem unmittelbar vorhergehenden Anspruch, wobei die Höhe des Zylinders so bestimmt ist, dass der Boden des Behälters an dem Deckel anliegt.

9. Vorrichtung nach einem der zwei unmittelbar vorhergehenden Ansprüche, wobei der Zylinder transparent ist, so dass der Behälter erkennbar ist.

10. Infusionsvorrichtung aufweisend einen Infusionsständer und eine Verabreichungsvorrichtung nach einem der vorhergehenden Ansprüche, die an dem Ständer aufgehängt ist.

11. Anordnung, aufweisend
- eine Verabreichungsvorrichtung nach einem der vorhergehenden Ansprüche, und
- mehrere Zylinder, die verschiedene Höhen und/oder verschiedene Innendurchmesser aufweisen und geeignet sind, sandwichartig zwischen den Sockel und den Deckel des Trägers gespannt sein zu können.

## Claims

1. Administration device comprising
- a hermetic container (12) closed by a cap (26), containing a radiopharmaceutical medicament (14), and provided with a protective shield made of a radiopaque material, and
- a support (16) provided with attachment means (30), the container (12) being removably attached to the support in a position allowing access to said cap,
**characterized in that** the support comprises a baseplate (22) provided with an opening (40) allowing access to said cap, a cover (20) and rods connecting the baseplate and the cover,
the container extending between the cover and the baseplate.

2. Device according to the preceding claim, wherein the protective shield comprises lead and/or tungsten.

3. Device according to either one of the preceding claims, wherein the attachment means comprise a ring.

4. Device according to any one of the preceding claims, wherein the cover (20) is provided with holes (32) through which the rods (24) pass, the rods being attached on the baseplate (22).

5. Device according to any one of the preceding claims, wherein the rods comprise stops (50) against which the cover bears by gravity.

6. Device according to any one of the preceding claims, wherein at least one, preferably each, rod (24) has, at its free end, a threaded portion (52) which protrudes above the cover and onto which a nut (54) is screwed so as to bear against the cover.

7. Device according to any one of the preceding claims, comprising a cylinder (60) resting on the support and in which the container is housed.

8. Device according to the immediately preceding claim, wherein the height of the cylinder is determined such that the bottom of the container bears against the cover.

9. Device according to either one of the two immediately preceding claims, wherein the cylinder is transparent, so that the container can be seen.

10. Perfusion apparatus comprising a perfusion stand and an administration device according to any one of the preceding claims, suspended from said stand.

11. Assembly comprising
- an administration device according to one of the preceding claims, and
- several cylinders having different heights and/or different inside diameters and which are adapted to be able to be sandwiched between the baseplate and the cover of the support.
